# EUROPEAN PATENT APPLICATION

(11) **EP 4 364 728 A1**
(43) Date of publication of application: **08.05.2024**
(21) Application number: 22833664.0
(22) Date of filing: 30.06.2022
(51) Int. Cl.: A61K 9/51, A61K 47/46, A61K 31/7088, A61K 48/00, A61K 9/00, A61K 45/06, A61P 35/00

(54) **PHARMACEUTICAL COMPOSITION CONTAINING CELL-DERIVED NATURAL OR ARTIFICIAL NANOVESICLES LOADED WITH ANTISENSE OLIGONUCLEOTIDE-BASED DRUG**

(30) Priority: 30.06.2021 KR 20210085716
(71) Applicant: Primoris Co., Ltd., Gwangmyeong-si, Gyeonggi-do 14322 (KR)
(72) Inventor: LEE, Jae-yong, Gwangju-si Gyeonggi-do 12777 (KR); AHN, Hee-jin, Seoul 02598 (KR); LEE, Dae-han, Seoul 08626 (KR); LEE, Dong-yeol, Seoul 06732 (KR); NA, Kyu-heum, Suwon-si Gyeonggi-do 16707 (KR)
(74) Representative: ZBM Patents - Zea, Barlocci & Markvardsen
(86) International application number: PCT/KR2022/009415
(87) International publication number: WO 2023/277610

(57) **Abstract**

The present invention relates to pharmaceutical compositions and methods of making the same, wherein nucleic acid-based drugs can be introduced into cells by using cell-derived natural or artificial nanovesicles loaded with nucleic acid-based drugs.

According to the invention, natural or artificial nanovesicles loaded with antisense oligonucleotides exhibit properties that inhibit proliferation and migration of cancer cells and increase drug sensitivity when co-administered with conventional chemotherapeutic agents.

## Description

### TECHNICAL FIELD

The present invention relates to pharmaceutical compositions comprising cell-derived natural or artificial nanovesicles loaded with nucleic acid-based drugs. For example, the present invention relates to the development of anticancer drugs utilizing human cell-derived natural or artificial nanovesicles loaded with antisense oligonucleotides against cancer-inducing gene sequences.

### BACKGROUND ART

Depending on the location of the cancer in the body, it may be called liver cancer, stomach cancer, uterine cancer, etc. Cancer in the lungs is called lung cancer, or more precisely, primary (lung-initiated) lung cancer, which is cancer that originates in the lung tissue, such as the bronchi, bronchioles, and alveoli. In contrast, cancer that originates in other organs and spreads to the lungs, such as breast or liver cancer, is called metastatic lung cancer and is treated differently from primary lung cancer.

Lung cancer is the most likely cause of death from cancer. It progresses slowly and can be treated surgically if detected early. Because of the different clinical course and treatment, lung cancer is microscopically divided into non-small cell lung cancer (NSCLS) and small cell lung cancer (SCLC) based on the size and shape of the cancer cells, with NSCLS accounting for 85% of lung cancer cases. NSCLC is further divided into non-squamous NSCLC and squamous NSCLC. Non-squamous NSCLC accounts for the majority of cases (70-75%), while squamous NSCLC accounts for about 25%. Despite the high therapeutic efficacy of some of the current targeted anti-cancer drugs for NSCLC, there is no treatment for resistant cancers caused by the EGFR T790M mutation.

All cells exchange information with other cells and the external environment. To do this, cells secrete a variety of substances into the environment, including growth factors, cytokines, chemokines, hormones, and neurotransmitters. In addition to these single substances, information exchange through extracellular vesicles (EVs) has gained increasing attention in recent years. In the case of bacterial exosomes, they may contain lipopolysaccharides or lipoteichoic acid.

Extracellular vesicles are nanoscale vesicles that all cells secrete into the external environment to exchange information between cells. They contain a variety of biologically active substances, including proteins, lipids, nucleic acids, and metabolites. Their composition reflects the type and condition of the cell from which they are derived, and they are found in a variety of body fluids, including cell cultures, blood, urine, saliva, tears, semen, breast milk, ascites, and cerebrospinal fluid. Extracellular vesicles derived from a variety of cells can interact with target cells to perform a variety of physiologic and pathologic functions. For example, stem cell-derived extracellular vesicles can induce tissue regeneration, while cancer cell-derived extracellular vesicles can affect cancer growth, metastasis, and angiogenesis. Bacterial-derived extracellular vesicles may have functions in antibiotic resistance, eliminating competing bacteria, delivering virulence factors, and modulating immune responses.

On the other hand, there are a number of proteins that are commonly found in extracellular vesicles from a variety of cells and fluids, indicating that proteins do not enter the extracellular vesicles in a chaotic manner, but through a regulated mechanism.

When the proteome for extracellular vesicles was analyzed according to the organelle of origin, proteins were mainly originated from the plasma membrane or cytosol, and relatively few proteins originated from mitochondria or the nucleus. The proteins found in common among extracellular vesicles are involved in the structure, production, and trafficking of extracellular vesicles, including tetraspanins (CD9, CD63, CD81, etc.), integrins, endosomal sorting complex proteins (TSG101, Alix), cytoskeletal proteins (actins, tubulin), and heat shock proteins (HSP70, HSP90). Proteins that are common to mammalian cell-derived extracellular vesicles can be used as labeling proteins to isolate extracellular vesicles. On the other hand, there are also cell type-specific proteins that are present on the extracellular vesicles, which are associated with performing cell type-specific physiological and pathological functions.

Because extracellular vesicles are composed of a variety of biologically active substances, they can perform a variety of biological functions. The mechanisms by which extracellular vesicles interact with target cells include ligand-receptor interactions, fusion, and endocytosis. Extracellular vesicles derived from various cells interact with target cells to perform physiological functions under normal circumstances or pathological functions that cause disease. As such, they can be utilized in the diagnosis and treatment of various diseases.

The various physiological functions performed by mammalian cell-derived extracellular vesicles include hemostasis, tissue regeneration, stem cell maintenance, regulation of inflammatory/immune responses, and embryonic development. Extracellular vesicles originating from various cells such as monocytes, endothelial cells, and platelets are present in blood vessels, and extracellular vesicles containing phosphatidylserine promotes blood clotting. In addition, extracellular vesicles derived from stem cells can mediate tissue regeneration and modulation of inflammatory responses in injury models such as kidney, liver, and skin. On the other hand, extracellular vesicles derived from immune cells can promote immune responses by delivering inflammatory cytokines such as IL-1β or directly or indirectly presenting antigens. They can also inhibit the immune response by delivering suppressive cytokines, such as TGF-β, or by inhibiting T cell activation. Extracellular vesicles with membrane-bound morphogens such as Wnt can bind to corresponding receptors to promote signaling pathways involved in embryonic development.

On the other hand, mammalian cell-derived extracellular vesicles have pathological functions that contribute to various diseases. In particular, in the tumor microenvironment, cancer cell-derived extracellular vesicles have a wide variety of functions. They promote angiogenesis by acting on endothelial cells via sphingomyelin to promote division, migration, and tube formation, and they can also differentiate monocytes into myeloid-derived suppressor cells. It also acts on other immune cells, inhibiting anti-cancer immunity by promoting the death of cytotoxic T cells or promoting the differentiation of regulatory T cells. This suggests that cancer cell-derived extracellular vesicles functions in the tumor microenvironment to promote cancer progression.

Nucleic acid drugs are medicines using nucleic acids, which function primarily as substances responsible for genetic information in vivo, such as siRNA, which inhibits gene expression, and aptamers, which specifically bind to target molecules.

Nucleic acid drugs are medicines based on nucleic acids (DNA or RNA), which are the building blocks of genes, and include antisense, siRNA, aptamers, and CpG oligomers. Since they work by acting on genes and proteins that cause diseases, they are expected to be effective and have fewer side effects because they can act on intracellular molecules that are difficult to target with existing medicines.

In particular, RNA-based therapeutics, such as small interfering RNAs (siRNAs), microRNAs (miRNAs), antisense oligonucleotides (ASOs), aptamers, synthetic mRNAs, and CRISPR Cas9, have significant potential to target many genes and gene products that are currently unregulated by drugs and create new therapeutic paradigms in diseases ranging from cancer to pandemic influenza, Alzheimer's, and autoimmune diseases. But before these RNAs can reach their full potential, they must overcome the long-established and evolutionarily perfected defenses that prevent RNAs from outside the cell from penetrating inside. Breaking through the lipid bilayer and delivering RNA into cells is a major challenge for the widespread development of RNA therapeutics, and a long-standing drug delivery problem.

Life arose when raw RNA and polymer solutions were encapsulated by lipid bilayers, allowing for independent chemical reactions without interference from outside RNA and macromolecules. The lipid bilayer allows for the passive diffusion of neutral, slightly hydrophobic molecules <1,000 Daltons (Da), while restricting the movement of large molecules such as RNA.

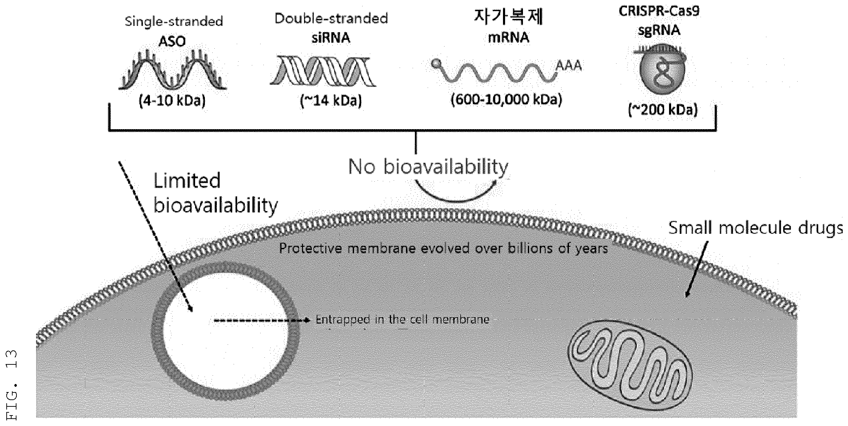

Thus, the lipid bilayer was fundamental in creating life and defending against invading RNA. On top of this ancient barrier are a series of evolutionary defenses designed to further protect metazoan cells from invading RNA, including RNases and the innate immune pattern recognition Toll like receptors (TLRs) 3, 7, 8, and the double helix RNA receptor PKR. In addition, RNA is rapidly removed from the blood by capture receptors on kidney and liver cells. Among these barriers, delivery across lipid bilayers remains a challenge. Small molecule inhibitors have a small molecular weight, low charge, and sufficient hydrophobicity to glide smoothly across the lipid bilayer of the cell membrane. In contrast, all RNA-based therapeutics do not have the ability to cross the lipid bilayer and are large, highly charged macromolecules (FIG. 13).

However, a 2017 Nature paper that found that exosomes significantly increased the therapeutic effectiveness of a gene therapy that inhibited the expression of KRAS, a gene involved in the development of pancreatic cancer, compared to liposomes, a traditional drug delivery vehicle, paved the way for further research and development of therapeutics using exosomes.

Extracellular vesicles, defined as biological membrane-enclosed structures that reside within a cell, vary widely in size and production. The term "exosome" refers to a cellular endosome that is released into the cell and ranges in size from 30 to 200 nm in diameter.

The process of exosome production is closely related to endosomes, the part of the cell that is responsible for absorbing and secreting substances. Late endosomes contain small vesicles called multivesicular bodies, which, when fused with the cell membrane, release their internal vesicles and become exosomes.

Exosomes have different properties depending on their size, biomarker proteins on their surface, function, and tissue of origin. In other words, exosomes are a generic term for biological membrane-enclosed structures found outside of cells that vary widely in size and characteristics.

The components of an exosome can be divided into the biological membrane and the components inside the biological membrane. The biological membrane, which separates the exosome from the outside world, contains various membrane proteins, lipids, and sugar chains, just like the components of the biological membrane inside the cell. Among the proteins present on the biological membrane of exosomes are several that are specific to exosomes and are considered biomarkers of exosomes, such as CD63, CD9, and CD81. The components inside exosomes include a variety of cell-derived proteins, RNA (miRNA, mRNA, etc.), and various metabolites.

Eventually, exosomes travel to other cells and exchange substances to transmit signals between cells. They play physiological and pathological roles by transmitting signals to other cells and affecting them.

Exosomes have various protein components in addition to the lipids that make up the biological membrane, and are naturally used as a means of exchanging substances between cells, allowing for more efficient mass transfer. They are also expected to be less immunogenic and toxic.

Virtually all mammalian cells are known to release exosomes. Ranging in size from 30 to 200 nm, exosomes are composed of a phospholipid bilayer with membrane proteins on the outside and contain proteins, mRNAs, miRNAs, and non-coding RNAs on the inside, meaning that their structure allows for the stable storage of genetic information.

Exosomes are biological nanoparticles ranging in size from 30 to 200 nm that are released by most cells and contain proteins and genetic information from the cell that releases them. Exosomes have been shown to be mediators of information transfer to neighboring or distant cells and regulate the microenvironment around cells.

Existing drug delivery systems are mainly composed of artificially synthesized materials, which have limited uptake by recipient cells and crossing the blood-brain barrier (BBB) due to biological heterogeneity. Exosomes, on the other hand, are produced by the body's own cells and have excellent biocompatibility and low cytotoxicity, so they do not cause immune rejection or other complications.

Exosomes have the advantage of easy internalization of drugs. Furthermore, exosomes can carry information through fusion with the cell membrane. In this way, genetic information and drugs delivered to cells can be degraded by the cell's digestive system.

### DETAILED DESCRIPTION OF THE INVENTION

### TECHNICAL PROBLEM

The present invention seeks to provide a method of developing an antisense oligonucleotide against Galectin-3, a cancer-promoting gene, into a cell-derived natural or artificial nanovesicle for use as an anticancer drug.

### TECHNICAL SOLUTION

The first aspect of the present invention provides a pharmaceutical composition comprising cell-derived natural or artificial nanovesicles loaded with an antisense oligonucleotide (ASO)-based drug.

A second aspect of the invention provides a pharmaceutical composition comprising cell-derived natural or artificial nanovesicles loaded with a Galectin-3 targeting nucleic acid drug.

A third aspect of the invention provides a pharmaceutical composition for oral or nasal inhalation or intravenous administration, comprising cell-derived natural or artificial nanovesicles loaded with a nucleic acid-based drug.

A fourth aspect of the invention provides a pharmaceutical composition for the prevention or treatment of cancer, comprising umbilical cord blood stem cell-derived natural or artificial nanovesicles loaded with a nucleic acid-based drug.

A fifth aspect of the invention provides a Galectin-3 targeting antisense oligonucleotide (ASO), which is designed using sequences within the Galectin-3 coding sequence (CDS).

Hereinafter, the present invention is described.

With regard to exosomal therapeutics, there are several methods for developing exosomes that closely resemble parent cells as therapeutic agents, and methods for utilizing exosomes in drug delivery systems (DDS). On the other hand, stem cells owe their ability to regenerate tissue to exosomes. Stem cell-derived exosomes have many advantages over stem cells as therapeutic agents. First of all, they are not living cells, which makes them easier to store and transport. They are also less likely to cause tumors and infections because they do not divide. If they are similar in efficacy, they are much easier to develop into therapeutics.

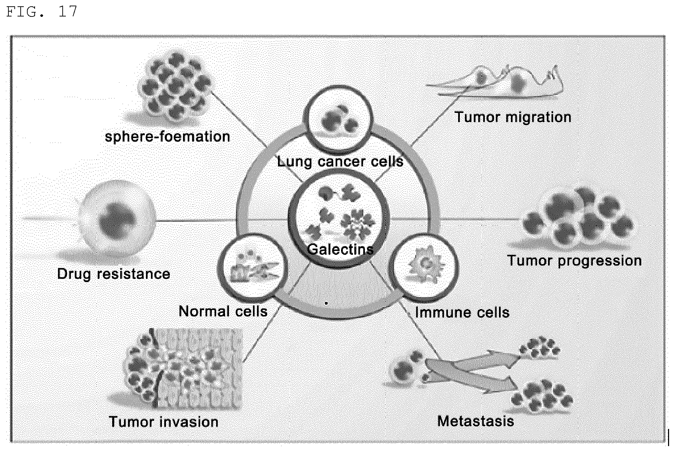

Galectin was isolated from animals in the 1970s and belongs to the lectin family. A total of 15 subtypes have been identified so far and are involved in various signaling processes in the cell by inducing cell-cell or cell-matrix interactions. The expression of galectins affects cell death, embryogenesis, inflammatory response, and cancer development. Among them, galectin-3 is highly expressed in NSCLC tissues and is known to promote the growth of cancer cells in cancerous tissues and to promote cancer metastasis by suppressing the immune response (FIG. 17).

Gene therapy typically involves introducing DNA fragments, miRNAs, siRNAs, and lncRNAs into target cells to reverse abnormal gene expression, induce the expression of suicide genes, modulate the immune response, and inhibit angiogenesis.

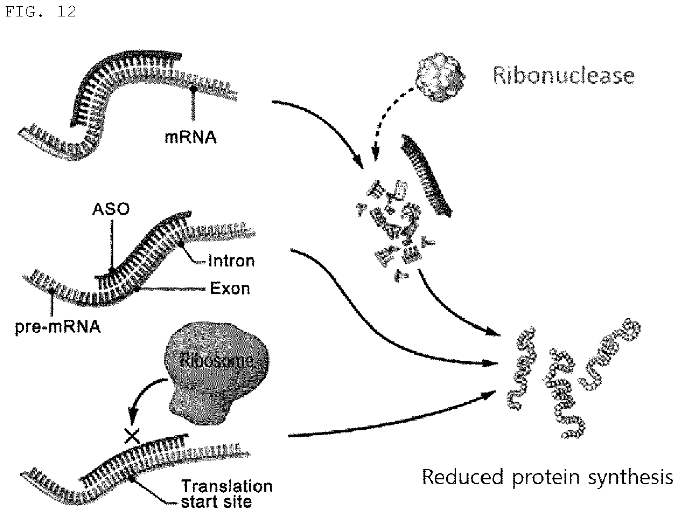

As shown in FIG. 12, antisense nucleotides have the advantage of being targetable, as they attach to the sequence of a specific gene and act as a mechanism to induce loss of function of the gene. However, as shown in FIG. 13, due to their single-stranded structure, they are not only unstable when treated alone, limiting their use as general-purpose drugs, but also cannot be delivered intracellularly in most tissues and cell types using current ASO delivery methods.

To solve the above problems, the present invention has confirmed that by loading these antisense nucleotides into exosomes, which are natural nanovesicles, or into artificial nanovesicles made by extruding and crushing cells, the stability of the drug is increased, and the effect of inhibiting the proliferation and migration of cancer cells is normally achieved. For example, the present invention developed a gene-targeting drug utilizing anti-sense oligonucleotide (ASO) and confirmed its effectiveness in suppressing the expression of Galectin-3, a gene involved in the proliferation and metastasis of non-small cell lung cancer. In other words, the function of Galectin-3 ASO as a drug to suppress cancer cell proliferation and metastasis was confirmed, and the possibility of loading Galectin-3 ASO into nanovesicles (including exosomes and artificial nanovesicles) was confirmed. Furthermore, we have confirmed that when co-administered with conventional anticancer drugs, it is effective in inducing the death of cancer cells by increasing their sensitivity. Therefore, the present invention not only enables the production of anticancer drugs utilizing antisense oligonucleotides loaded into nanovesicles, but also enables them to be used in combination with existing anticancer drugs.

In short, a feature of the present invention is the use of cell-derived nanovesicles instead of viral vectors as a nucleic acid delivery system and/or the use of various nucleic acid-based drugs, especially ASO-based drugs, with high bioavailability. By loading nucleic acid-based drugs onto cell-derived natural or artificial nanovesicles, the nucleic acid-based drug 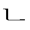 can be distributed into the intracellular fluid. Furthermore, the use of cell-derived nanovesicles as a tool for the delivery of therapeutic nucleic acids to target cells in accordance with the present invention may improve therapeutic efficacy and avoid side effects.

In the present invention, cell-derived natural nanovesicles as well as artificial nanovesicles can be used as nucleic acid delivery systems.

Since reliability, reproducibility, donor-donor variations, and reflection of intrinsic properties of parental cells are important concerns in cell-derived nanovesicle formulations, it is preferable to use umbilical cord blood stem cell-derived natural or artificial nanovesicles when stem cells are utilized as parental cells, and when the intrinsic properties of stem cells, such as promotion of regeneration, affect the proliferation of cancer cells, it is preferable to utilize cells other than stem cells that are appropriate for the disease to be treated.

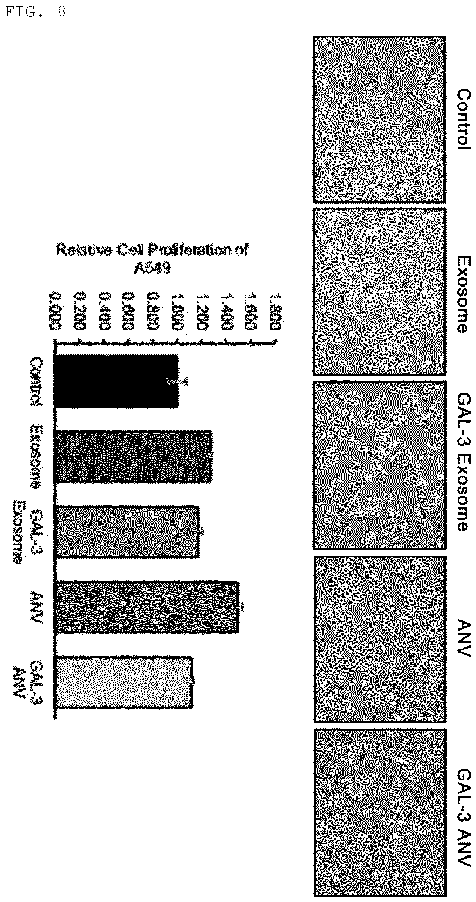

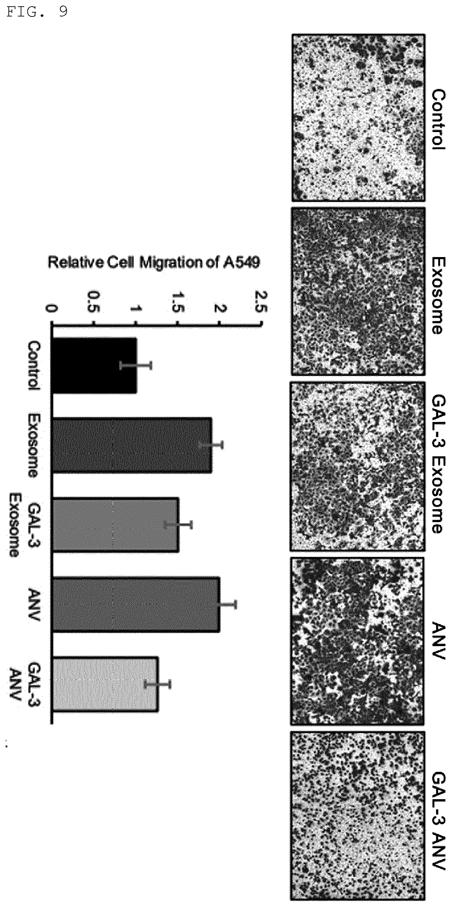

Stem cell exosomes themselves have the ability to promote the proliferation and migration of other cells, and thus also promote the proliferation and migration of cancer cells. Nevertheless, surprisingly, we found that while treatment with umbilical cord blood stem cell-derived exosomes alone increased the proliferation and migration of cancer cells, co-administration of umbilical cord blood stem cell-derived exosomes with anticancer agents that inhibit the proliferation and migration of cancer cells reduced the proliferation and migration of cancer cells (FIGS. 8 and 9).

It is inferred that the anti-inflammatory substances contained in exosomes derived from umbilical cord blood stem cells are associated with five times the amount of exosomes derived from other tissues. This is because the inflammatory substances released from inflammatory cells are closely related to the proliferation, survival, and metastasis induction of cancer cells, and inflammation in the tumor microenvironment has a tumor-promoting effect, not only helping cancer cells survive and proliferate, but also promoting angiogenesis and metastasis, disrupting the adaptive immune response, and altering their responsiveness to hormones and drugs.

Therefore, based on the above findings, according to the present invention, umbilical cord blood stem cell-derived natural or artificial nanovesicles can be used in pharmaceutical compositions for the prevention or treatment of cancer, and can be used as drug delivery systems (DDS) for the prevention or treatment of cancer.

As used herein, cell-derived natural nanovesicles are referred to interchangeably as extracellular vesicles or exosomes (extracellular vesicles ranging in size from 30 to 200 nm).

### [Extracellular Vesicles]

Extracellular vesicles are nanoscale vesicles surrounded by a lipid bilayer that all cells release into the external environment to exchange information between cells.

Because they have a membrane and lumen and the same membrane topology as cells, they can be genetically engineered to express various membrane proteins and deliver drugs to target cells or tissues with relative accuracy. In addition, various drugs can be loaded into both the membrane and lumen of the extracellular vesicle. When drugs are loaded into the lumen, they can be safely delivered to the target cell. Since they have the same membrane as cells, drug delivery in the lumen is possible through membrane fusion.

One of the biggest advantages in the therapeutic use of extracellular vesicles comes from their low-grade immunogenicity. Because extracellular vesicles are derived from cells, they have low immunogenicity. For example, extracellular vesicles derived from mesenchymal stem cells (MSCs) are known to carry a very low risk of immunogenicity. Therefore, they contribute to a longer lifespan and higher bioavailability of the loaded drug. In addition, their size allows them to cross the blood brain barrier, allowing them to be transported to all tissues in the body.

Extracellular vesicles contain a variety of biologically active substances, including proteins, lipids, nucleic acids, and metabolites, and their composition varies depending on the type and condition of the cells from which they are derived. While some components are common to all extracellular vesicles, others are cell type specific.

Mammalian cell-derived extracellular vesicles contain higher levels of phosphatidylserine, cholesterol, sphingomyelin, and GM3 ganglioside compared to their cell of origin. The lipids present in extracellular vesicles contribute to their formation, stability, and function. Phosphatidylserine is involved in the formation of extracellular vesicles, while cholesterol, sphingomyelin, and GM3 gangliosides collectively increase the stability of extracellular vesicles. In addition, cancer cell-derived extracellular vesicles have been shown to promote angiogenesis through sphingomyelin.

In addition to mRNAs and miRNAs, rRNAs, tRNAs, and DNAs are also known to be present in extracellular vesicles. While some RNAs are present in similar amounts to their cell of origin, there are certain RNAs that are more abundant in extracellular vesicles than in their cell of origin, suggesting that RNAs, like proteins, are regulated by the same mechanisms that lead to their organization in extracellular vesicles.

Because extracellular vesicles perform physiological functions and can be targeted for delivery to specific cells or tissues, they can be used as disease therapeutics, drug delivery, and vaccines.

Because extracellular vesicles are not living cells, they can be stored and transported for long periods of time. And because they do not divide, mammalian-derived extracellular vesicles are less likely to be tumorigenic.

Extracellular vesicle uptake is highly efficient due to membrane proteins such as tetraspanins, fibronectin, and proteoglycans, but can also be customized according to the target cell.

Extracellular vesicles enriched with vascular cell adhesion molecule 1 and integrin α4 enhance nanovesicle docking and uptake by endothelial cells.

Due to their small size, extracellular vesicles can cross the blood-brain barrier in a bidirectional manner through transcytosis (endocytosis followed by multivesicular body formation and release on the other side) or through junctions between endothelial cells.

The innate and unique properties of the extracellular vesicles in cell-cell communication make them useful as a nucleic acid delivery system. Extracellular vesicle-mediated transport appears to be highly effective, allowing for higher doses of therapeutic agents to be released into the tumor, reducing the amount in the blood and reducing side effects. In addition to delivering therapeutics, extracellular vesicles have functional utility. They can signal through a variety of modalities by activating receptors on the cell surface, delivering their contents inside the cytoplasm, delivering hydrophilic molecules inside, lipophilic molecules in the lipid bilayer, and amphiphilic molecules on the surface.

### [Prepare cell-derived natural or artificial nanovesicles (exosomes + artificial nanovesicles)].

Natural or artificial nanovesicles can be released or prepared from cells, respectively.

Nanovesicle is a description of a pocket-like substance with a diameter of nanometers. A typical nanovesicle is an exosome, which is gaining attention as a biocompatible drug delivery system that can replace cellular therapeutics.

The amount of naturally released exosomes is limited, and their productivity is low. Approaches include developing isolation and purification techniques to increase the yield of exosomes, or using different culture conditions to increase the rate of releasing from cells.

Artificial nanovesicles are exosomes made artificially from cells and are much more productive than naturally secreted exosomes.

Methods for isolating exosomes include ultracentrifuge, ultrafiltration, and chromatography, and the functional properties, purity, concentration, and size of exosomes vary depending on the isolation method.

Exosomes released from adult stem cells have been shown to have excellent regenerative and anti-inflammatory effects on surrounding cells.

Large-scale production of nanovesicle-based formulations is critical to the success of clinical translation. However, since the amount of naturally released extracellular vesicles is limited, therapies using artificial nanovesicles as extracellular vesicles mimics have been attempted. Artificial nanovesicles are made by crushing the cell itself into exosomes, which solves the biggest disadvantage of exosomes, which is the problem of mass production, and has the advantage of efficiently loading drugs during the nanovesicle formation process.

Methods for producing artificial nanovesicles include serial extrusion or repetitive extrusion utilizing a porous membrane. In addition, the present invention can produce artificial nanovesicles by utilizing a high pressure homogenizer or a nano disperser.

Normally, small cells are around 20 um in diameter and have limited mobility in the body. However, exosomes have the advantage of being able to pass through the blood-brain barrier due to their inherent small size, allowing them to deliver their active ingredients to all parts of the body. Therefore, utilizing the cells themselves and crushing them to exosome size provides the advantage of reaching all parts of the body while containing a high content of intracellular active ingredients.

Furthermore, cells, the basic structural and activity unit of an organism, can be cultured in vitro and used in pharmaceutical, tissue engineering, and therapeutic development. Cells have the ability to adapt to their surroundings, so even within the same cell species, the adhesion, growth, migration, and differentiation of cells can vary depending on the culture environment. Furthermore, stem cells, which have recently received a great deal of attention in the field of regenerative medicine, can be induced to differentiate into desired cells and grow by applying appropriate stimuli in culture. Furthermore, the artificial nanovesicles contain various molecular components (e.g., proteins and RNA) of the cell from which the artificial nanovesicles are derived. Thus, the protein composition of the artificial nanovesicles can be varied depending on the culture conditions of the cells from which the artificial nanovesicles are derived (e.g., the molecular signals received by the cells during culture).

For example, artificial nanovesicles produced from stem cells according to the present invention can provide nanosized artificial nanovesicles with a unique lipid bilayer structure in large quantities, and can exert tissue regeneration and anti-aging effects, increased collagen synthesis, and wound healing effects through proliferation and activation of fibroblasts because they contain a large amount of various growth factors in the artificial nanovesicles.

### [Drug Loading Methods]

There are three main ways to load drugs into nanovesicles.

The first is to utilize an ultracentrifuge, which induces the drug in the solvent to flow into the vesicle by centrifugal force.

The second is electroporation, which is a method in which the charge on the outside of the membrane becomes negative and the charge on the inside of the membrane becomes positive for a short period of time, causing a hole in the membrane, and the drug enters the vesicles through the hole.

The third method utilizes a method in which drugs suspended in suspension are naturally incorporated into the vesicle spheres as the cells are crushed and reformed into spheres.

The yield of artificial nanovesicles is much higher than that of naturally released extracellular vesicles, and the drug delivery efficiency is similar.

### [Modification of Single Stranded Anti-Sense Oligonucleotide (ASO)]

In the present invention, an anti-sense oligonucleotide (ASO)-based drug includes an ASO-based drug in which some or all of the bases of at least one of the sequences are modified with phosphorothioate and/or 2'-O-methyl groups.

As shown in FIG. 12, ASOs work by targeting the mRNA sequence of a particular gene with a DNA sequence of ASOs to induce degradation of that site so that the translation process cannot proceed, resulting in loss of function. Since its discovery in 1978, four drugs based on ASO have been approved by the U.S. FDA. However, the nucleotide structure of 8 to 50 base pairs is not very stable in the body, which is a disadvantage in terms of efficacy and utilization as a drug.

The field of single-stranded ASOs began in 1978 with the synthesis of ASOs with a phosphodiester backbone. Unfortunately, phosphodiester bonds are highly charged, hydrophilic, and easily cleaved by nucleases. Modification to a mononucleotide (phosphorothioate) in which the oxygen of one of the phosphate groups is replaced by a sulfur atom can greatly increase resistance to phosphodiesterases and increase hydrophobicity. In 1984, the first fully phosphorothioate ASO was synthesized, dramatically improving its stability and deliverability.

To improve the efficacy and pharmacological properties of ASOs, the 2' hydroxyl (OH) can be chemically modified. This can increase the binding affinity to the target mRNA and thereby reduce the overall length of the ASO.

Two alternative antisense compound approaches are to replace the charged phosphate bond and ribose glycosidic backbone with a neutral phosphorodiamidate morpholino oligomer (PMO) or peptide nucleic acid (PNA) backbone to maintain the correct nuclear base spacing. The result is a highly stabilized molecule that binds selectively and strongly to the target mRNA. However, because PMOs and PNAs lack a phosphodiester backbone, they cannot activate RNase H (which is required for gene silencing) and can instead be used as steric blockers and SSOs for exon skipping.

### [Nucleic acid-based drugs]

In addition to the ASOs described above, the nucleic acid-based drugs loaded into the cell-derived natural or artificial nanovesicles of the present invention can be mRNAs, miRNAs, rRNAs, tRNAs, and DNAs.

Compared to ASOs and miRNAs, it is difficult to enclose large mRNAs in exosomes. However, in the case of artificial nanovesicles, the size of the vesicles can be easily controlled, so it is relatively easy to load mRNA into the vesicles.

As used herein, nucleic acid-based drugs include naturally or artificially synthesized nucleic acids as well as modified nucleic acids.

The mRNAs delivered via the cell-derived natural or artificial nanovesicles of the present disclosure may increase the expression of the proteins encoded by those mRNAs in a target cell, or, if miRNAs are delivered, may decrease the expression of the mRNAs targeted by those miRNAs in a target cell.

Due to their high selective specificity for target RNA or DNA, RNA-based therapies can selectively inhibit the expression of drug-resistance human and viral genes, regulate the splicing of mRNAs, target non-coding RNAs (ncRNAs) involved in epigenetic regulation, increase the number of target genes, express genes, and modify the genome. These are modes of therapeutic action that cannot be realized through small molecule inhibitors or antibodies. RNA-based therapies are also the only ones with the ability to evolve to keep pace with cancer mutations and infectious disease viral infections. Second-generation RNA chemistry technologies greatly improve the stability of RNA therapeutics, reduce unintended side effects, and maximize pharmacological activity on target.

### [Types of loaded drugs (nucleic acid-based drugs and conventional anticancer drugs)].

In addition to the nucleic acid-based drugs described above, the types of drugs that can be loaded into nanovesicles are not limited. Along with conventional chemotherapeutic agents such as cisplatin and 5-FU, ASOs targeting other cancer-related genes such as Galectin-3 are also possible. Specific gene-targeting siRNAs, which have already been extensively tested ex vivo, can also be utilized. In addition, a family of miRNAs that regulate gene expression can be loaded.

For example, exosomes can be artificially loaded with short interfering RNAs (siRNAs) or miRNAs to suppress the expression of specific genes and then used as drug delivery vehicles. For example, an siRNA targeting the cancer gene KRAS (G12D) can be introduced by electroporation into mesenchymal stem cell-derived exosomes.

To deliver exosomes specifically to a particular cell or place a specific mRNA within an exosome, exosomes can be created based on genetically engineered cells. For example, to deliver exosomes specifically to breast cancer cells, a protein fused with a peptide that binds to integrin αV, a marker for breast cancer cells, can be put into an exosome; to deliver a drug that can kill cancer cells, such as doxorubicin, into an exosome; or to package mRNA into an exosome, an RNA binding portion can be fused to CD63, an exosome marker protein, to deliver specific mRNA through an exosome.

### [Cellular origin of nanovesicles and Umbilical cord blood stem cells]

The cellular origins of the natural or artificial nanovesicles of the present invention range from dendritic cells, reticulocytes, erythrocytes, monocytes, and macrophages to cell lines capable of permanent lineage through genetic manipulation(e.g., HEK293 cells), including mesenchymal stem cells (MSCs) and human iPSCs. MSCs are the primary source for the production of natural or artificial nanovesicles of the present invention for clinical trials. MSC-derived extracellular vesicles can be easily scalable and safe for patients.

Types of stem cells include embryonic stem cells, adult stem cells, and reverse differentiated stem cells, of which adult stem cells are derived from blood, bone marrow, fat, etc. and are free from ethical issues unlike embryonic stem cells.

Adult stem cells are undifferentiated cells that will differentiate into cells of a specific tissue when needed. Adult stem cells can be, but are not limited to, mesenchymal stem cells, mesenchymal stromal cells, or pluripotent stem cells.

Mesenchymal stem cells play an important role in regeneration by releasing various growth factors and cytokines, such as epithelial growth factor and fibroblast growth factor, to stimulate the production of collagen, fibronectin, and elastin from fibroblasts.

Mesenchymal stem cells (MSCs) are progenitor cells that have the ability to differentiate into neurons, adipocytes, chondrocytes, osteoblasts, muscle cells, cardiac tissue, and other endothelial or epithelial cells. These cells can be phenotypically defined by gene or protein expression. Thus, MSCs can be characterized phenotypically and/or functionally based on their differentiation potential. MSCs can be collected from a number of sources, including but not limited to bone marrow, blood, periosteum, dermis, umbilical cord blood and/or matrix (e.g., Wharton's jelly), and placenta. MSCs can be isolated from multiple sources, e.g., bone marrow mononuclear cells, umbilical cord blood, adipose tissue, and placental tissue, based on their adherence to cell culture dish.

Unlike embryonic stem cells derived from human embryos, adult stem cells are derived from already grown body tissues, such as bone marrow or brain cells, and thus have the advantage of avoiding ethical controversies. As used herein, adult stem cells may be derived from, but are not limited to, umbilical cord, umbilical cord blood, bone marrow, fat, muscle, nerve, skin, amniotic membrane, or placenta.

Exosomes produced by mesenchymal stem cells or dendritic cells have physiological activities of their own and are being explored for therapeutic purposes.

In particular, the activity of mesenchymal stem cells in repairing or regenerating damaged tissues is believed to be due to growth factors delivered by exosomes from mesenchymal stem cells rather than mesenchymal stem cells themselves. There have been reports that exosomes derived from MSCs are effective in treating graft-versus-host diseases, a side effect of hematopoietic stem cell transplantation.

While bone marrow- or adipose-derived stem cells are extracted from a donor using invasive methods, umbilical cord blood stem cells are extracted from umbilical cord blood discarded after childbirth, which has the advantage of not being invasive to the donor and having small differences in umbilical cord blood stem cell potency between donors.

If stem cells are utilized to produce the natural or artificial nanovesicles of the present invention, they may be released or prepared from umbilical cord blood stem cells. Unlike adipose- or bone marrow-derived adult stem cells, umbilical cord blood-derived stem cells have the advantage that they are not subject to differences in efficacy based on the donor's condition, since the umbilical cord blood is formed during the donor's pregnancy cycle (40 weeks).

Umbilical cord blood mesenchymal stem cells (UCB-MSCs) contain a variety of growth factors, including EGF, VEGF, TGF, HGF, FGF, IGF, and PDGF. Therefore, growth factors such as EGF, which are loaded in artificial nano-vesicles produced from umbilical cord blood mesenchymal stem cells, promote fibroblast proliferation, cell migration, and collagen synthesis.

Among the tissues from which adult stem cells can be obtained (fat, bone marrow, umbilical cord blood), exosomes isolated from umbilical cord blood contain the most regenerative and anti-inflammatory factors. Exosomes derived from umbilical cord blood stem cells contain five times the amount of anti-inflammatory substances as exosomes from other tissues.

### [Formulation of nucleic acid-based drug-loaded nanovesicles].

Nanovesicles produced according to the present invention can be formulated in various ways depending on the intended use. The formulation of cell-derived natural or artificial nanovesicles loaded with nucleic acid-based drugs according to the present invention can be concentrated, gelled, lyophilized, and reconstituted in aqueous solutions without significantly altering their properties.

Drug nanoformulations hold incredible promise for efficient delivery of therapeutics to the site of disease. Unfortunately, artificial nanocarriers, mostly liposomes and polymeric nanoparticles, show limited application due to unfavorable pharmacokinetics and rapid clearance from the blood circulation by the reticuloendothelial system (RES). Moreover, many of them suffer from high cytotoxicity, low biodegradability, and inability to cross biological barriers including the blood-brain barrier.

The cell-derived natural or artificial nanovesicles used as drug delivery systems in the present invention have high bioavailability, excellent biocompatibility, and low immunogenicity.

The cell-derived natural or artificial nanovesicles used as drug delivery systems in the present invention are capable of delivering nucleic acid-based drugs loaded thereon to target tissues, cells, and organs. Similar to artificial nanocarriers, the cell-derived natural or artificial nanovesicles of the present invention can improve the basic properties of the free nucleic acid-based drug, such as stability and solubility, and protect the nucleic acid-based drug from degradation in the bloodstream. They can also provide protection from extracellular nucleases.

The membrane of the cell-derived natural or artificial nanovesicles of the present invention is a relatively rigid lipid bilayer, which can provide sustained and prolonged release of the incorporated nucleic acid-based drug. Furthermore, unlike most synthetic nanocarriers, the cell-derived natural or artificial nanovesicles of the present invention can cross biological barriers, including the blood-brain barrier (BBB), making them particularly useful in the treatment of neurodegenerative diseases.

Furthermore, the cell-derived natural or artificial nanovesicles of the present invention have low immunogenicity and low cytotoxicity. Therefore, the cell-derived natural or artificial nanovesicles of the present invention are also preferably prepared from isolated autologous cells.

The cell-derived natural or artificial nanovesicles of the present invention can be designed to exhibit tissue specificity to enable migration to specific cell types or inflamed tissues. The natural or artificial nanovesicles of the present invention may have intrinsic biological activities that reflect their origin, i.e., the parent cell, which may provide additional therapeutic efficacy to the incorporated nucleic acid-based drug.

The biological activities of natural or artificial nanovesicles released/prepared from different types of cells are vast and promising. However, since reliability, reproducibility and donor-donor variations in formulation are important, it is desirable that the cell-derived natural or artificial nanovesicles of the present invention are prepared in accordance with the characteristics of the disease to be treated.

### [Alveolar structure and route of administration]

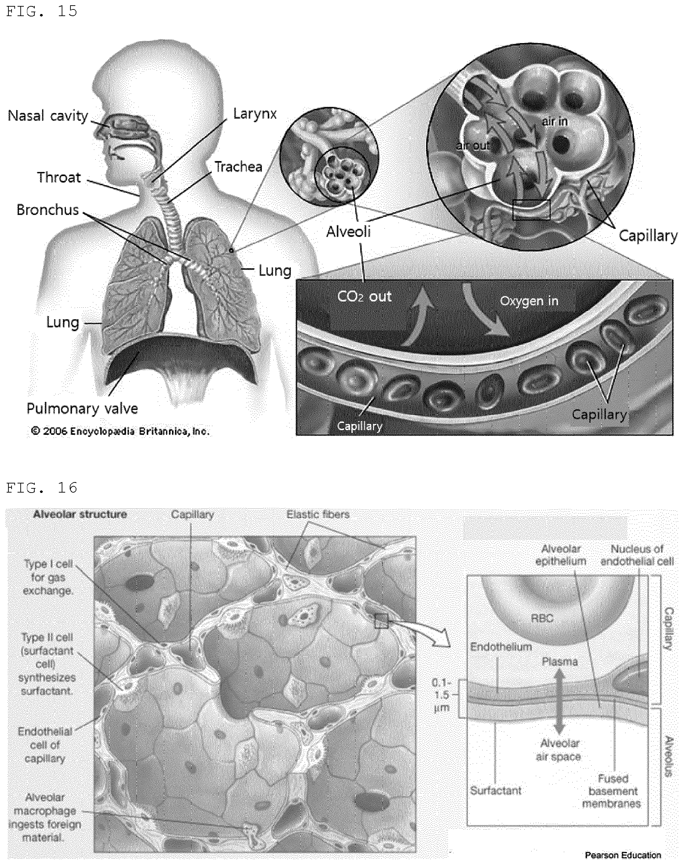

As shown in FIG. 15, the primary function of the respiratory system is to deliver oxygen by pumping oxygenated blood throughout the body. Respiration occurs through the mouth, nose, trachea, lungs, and diaphragm. First, oxygen enters the respiratory system through the nose and mouth, and then through the larynx and trachea into the chest cavity. Inside the chest cavity, the trachea divides into two smaller tubes called bronchi. Each bronchus divides further, forming a tree-like branch. The bronchi, which go directly into the lungs, further divide into many bronchioles, which connect at their ends to tiny sacs called alveoli.

An adult male has about 6 million alveoli, which are air sacs surrounded by capillaries. The total cross-sectional area of the alveoli is 70 to 90 m², which is about 50 times the total area of the body's skin.

FIG. 16 shows the structure of alveoli. Pulmonary surfactant, a combination of phospholipids and proteins secreted by Type II alveolar cells, reduces surface tension by weakening the hydrogen bonds between water molecules, so that surface tension usually does not cause alveolar collapse.

On the other hand, the function of epithelial tissue is to line the interior and surface of the organ. Epithelial tissue is a dense arrangement of lamellar cells in a single or multiple layers, and it lines the inside of organs or hollow organs or body cavities. Epithelial tissues always have an apical surface that is exposed to the outside of the body or the inside of a body cavity, and a basal surface that is connected to other tissues by a basement membrane, a noncellular substance.

Epithelial tissue provides a covering that separates one tissue from another and separates the body from the external environment. The structure of epithelial tissue is closely related to its function.

For example, the lining of the heart and blood vessels, the alveoli of the lungs, and the glomeruli of the kidneys are monolayer squamous epithelium with a monolayer arrangement of flat cells that perform filtration and diffusion functions, and the alveolar epithelium of the lungs is very thin to facilitate gas exchange (FIG. 16).

Thus, as shown in FIG. 16, cell-derived natural or artificial nanovesicles loaded with nucleic acid-based drugs according to the present invention can be delivered directly to the alveoli through the trachea (airways) by inhalation into the mouth/nose, or can be delivered to the alveoli through capillaries after intravenous administration.

### [inhibit the ability of target cells to proliferate and/or migrate].

Cancer is a group of abnormal cells (cancer cells) that develop when normal cells in the body mutate. Cancer cells are out of control of the normal regulatory mechanisms in the human body and proliferate in a disorderly manner, metastasizing to other organs (normal tissues) and repeating their proliferation, eventually damaging the function of those organs (tissues).

According to the present invention, cell-derived natural or artificial nanovesicles loaded with nucleic acid-based drugs can reduce the proliferative and/or migratory capacity of target cells, especially cancer cells, and can be used to intensively prevent or treat lung cancer, especially non-small cell lung cancer, via the oral or nasal route of administration, inhalation or intravenous injection.

### [Inhalation through the mouth or nasal passages]

Inhalation or inspiration occurs when air or other gases enter the lungs.

Inhalation through the mouth or nasal cavity results in rapid absorption of the drug through the large surface area of the airway mucosa and lung epithelium, with a rapid onset of action almost identical to that of intravenous injection. This route minimizes systemic side effects as cell-derived natural or artificial nanovesicles loaded with nucleic acid-based drugs according to the present invention are transported locally and directly to the site of action. Furthermore, the inhalation route of administration allows for rapid absorption and immediate onset of effect, which allows for dose titration and localized effects targeting the lungs. Thus, lower doses can be used compared to oral or parenteral doses.

Thus, cell-derived natural or artificial nanovesicles loaded with nucleic acid-based drugs according to the invention can be formulated in the gaseous state or dispersed in an aerosol.

When administered by inhalation through the mouth, they should be dispersed in smaller droplets than when administered via the nasal route so that they can be delivered to the lungs via the trachea (airways). The depth of delivery to the lungs depends on the size of the droplets. The smaller the droplet, the deeper it is delivered and the greater the amount absorbed. In this case, cell-derived natural or artificial nanovesicles loaded with nucleic acid-based drugs according to the present invention can enter the bloodstream from inside the lungs.

Relatively few drugs are administered in this way because inhalation must be carefully monitored to ensure that the proper amount is administered within a specified time frame. In addition, specialized equipment may be required to administer drugs by the inhalation route. In order to deliver a dose that acts specifically on the lungs, an aerosolized metered dose container (called an inhaler) can be used, or a gaseous administration method can be used.

On the other hand, similar to the inhalation route, when administered by nebulization, it must be delivered in small particles to reach the lungs. Nebulization can be accomplished using specialized devices, most commonly ultrasonic or jet nebulizer systems. Proper use of the device helps to maximize the amount of drug delivered to the lungs.

### [Administration via intravenous injection]

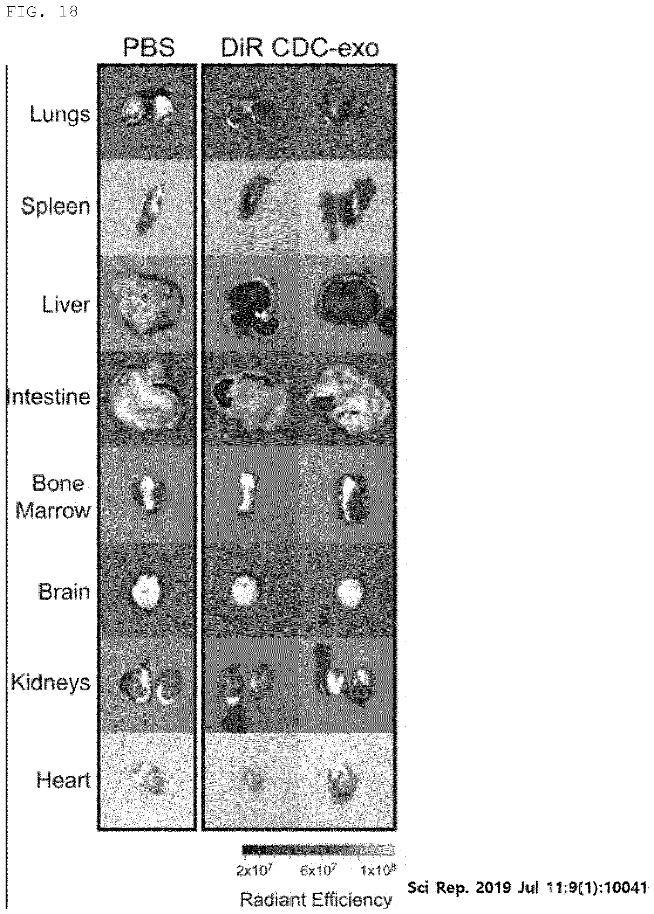

Previous studies have confirmed that extracellular vesicles injected into the body via intravenous injection circulate in the body along the bloodstream, reaching and residing in the liver and lungs (FIG. 18). Therefore, it is possible to induce the effect of delivering a substance inside the extracellular vesicles to the lung tissue by utilizing a mechanism that naturally acts on the lungs without any specific manipulation of the exterior of the vesicles and without conferring a targeting function to a specific tissue. Thus, lung cancer therapeutics comprising cell-derived natural or artificial nanovesicles loaded with nucleic acid-based drugs in accordance with the present invention can utilize exosomes to address the limitation that drugs act systemically, resulting in reduced therapeutic effectiveness and serious side effects.

### [pharmaceutical composition]

The pharmaceutical composition may further comprise a pharmaceutically acceptable concentration of a salt, a buffer, a preservative, a compatible carrier, and/or another (i.e., secondary) therapeutic agent.

Each component of the pharmaceutical composition should be in a pharmaceutically acceptable form.

A pharmaceutically acceptable carrier is a pharmaceutically acceptable substance, composition, or vehicle, such as a liquid or solid filler, diluent, excipient, solvent, or encapsulating material, that is involved in carrying or transporting a prophylactically or therapeutically active agent. Each carrier must be "acceptable" in that it is compatible with the other components of the formulation and is not harmful to the subject. Some examples of substances that can serve as pharmaceutically acceptable carriers are sugars, such as lactose, glucose, and sucrose; glycols, such as propylene glycol; polyols, such as glycerin, sorbitol, mannitol, and polyethylene glycol; esters, such as ethyl oleate and ethyl laurate; buffers, such as magnesium hydroxide and aluminum hydroxide; distilled water for injection; isotonic saline; Ringer's solution; ethyl alcohol; phosphate buffer solutions; and other non-toxic compatible materials utilized in pharmaceutical formulations.

In the present disclosure, the pharmaceutical composition may be formulated for the treatment of a lung disease. Accordingly, the pharmaceutical composition may further comprise other therapeutic agents (secondary agents) that may be used to treat the lung disease. As used herein, a therapeutic agent refers to any agent that can be used to prevent, treat, and/or manage a pulmonary disease such as those discussed herein. These include, but are not limited to, surfactants, inhaled nitric oxide, almitrine bismesylate, immunomodulators, and antioxidants. Examples of immunomodulators include steroids and corticosteroids, such as, but not limited to, methylprednisolone. Examples of antioxidants include, but are not limited to, superoxide dismutase.

For the treatment of lung disease, the second agent may be a pulmonary surfactant. A "pulmonary surfactant" is a lipoprotein mixture that is useful in keeping the pulmonary airways open (e.g., by preventing alveolar walls from adhering to each other). The pulmonary surfactant may comprise phospholipids, such as dipalmitoylphosphatidylcholine (DPPC), phosphatidylcholine (PC), phosphatidylglycerol (PG); cholesterol; and proteins, such as SPA, B, C, and D. The pulmonary surfactant may be derived from a naturally occurring source, such as bovine or porcine lung tissue. Examples include Alveofact^{™} (from bovine lung lavage fluid), Curosurf^{™} (from chopped porcine lung), Infasurf^{™} (from calf lung lavage fluid), and Survanta^{™} (from chopped bovine lung; with additional ingredients including DPPC, palmitic acid, and tripalmitin). Lung surfactants can also be synthetic. Examples include Exosurf^{™} (composed of DPPC in combination with hexadecanol and thyroxapol), Pumactant^{™} or artificial lung expansion compound (ALEC) (composed of DPPC and PG), KL-4 (DPPC, palmitoyl-oleoyl phosphatidylglycerol, palmitic acid, and a synthetic peptide mimicking SP-B), and Venticute^{™} (composed of DPPC, PG, palmitic acid, and recombinant SP-C). pulmonary surfactants may be obtained from commercial suppliers.

An "effective amount" is the amount of an agent that achieves a desired outcome. The absolute amount will depend on a variety of factors, including the material selected for administration, whether the administration is a single dose or multiple doses, and individual patient parameters including age, physical condition, size, weight, and stage of disease. These factors are well known to those of ordinary skill in the art and can be addressed by simply routine experimentation.

In one embodiment, an effective amount is a dose of an agent that does not cause toxicity in a subject. In one embodiment, an effective amount is a dose of an agent that reduces toxicity to the subject. Methods for measuring toxicity are well known in the relevant art (e.g., biopsy/histology of liver, spleen, and/or kidney; alanine transferase, alkaline phosphatase, and bilirubin tests for liver toxicity; and creatinine levels for kidney toxicity).

The effective dose level may be determined based on factors including the type and severity of the individual, age, gender, type of disease, activity of the drug, sensitivity to the drug, time of administration, route of administration and rate of elimination, duration of treatment, concomitant medications, and other factors well known in the medical field. In addition, the effective amount may vary depending on the route of treatment, the use of excipients, and the potential for use with other agents, as recognized by those skilled in the art.

"Treat" or "cure" includes, but is not limited to, preventing, reducing or stopping the occurrence of a disease, reducing or eliminating the symptoms of a disease, or preventing a disease.

A subject may be a human or vertebrate animal or mammal, including, but not limited to, a rodent, e.g., rat or mouse, dog, cat, horse, cow, pig, sheep, goat, turkey, chicken, and a primate, e.g., monkey. The methods of the present disclosure are useful for treating a subject in need of treatment. The subject in need of treatment can be a subject at risk of developing a disease (e.g., via genetic testing) or a subject with a disease.

In one embodiment, a composition comprising cell-derived natural or artificial nanovesicles loaded with a nucleic acid-based drug is administered to a subject in a bolus dose. A "bolus dose" refers to a single administration of a discrete amount of a medicament, drug, or other compound to achieve a therapeutically effective level. In one embodiment, repeated administration of said nucleic acid-based drug-loaded nanovesicles is contemplated, including two, three, four, five or more doses of said nucleic acid-based drug-loaded nanovesicles. In some cases, the nucleic acid-based drug-loaded nanovesicles may be administered consecutively. Repeated or continuous administration may occur over a period of hours (e.g., 1-2, 1-3, 1-6, 1-12, 1-18, or 1-24 hours), days (e.g., 1-2, 1-3, 1-4, 1-5, 1-6, or 1-7 days), or weeks (e.g., 1-2 weeks, 1-3 weeks, or 1-4 weeks), depending on the severity of the condition being treated. If dosing is repeated but not consecutive, the time between doses may be several hours (e.g., 4 hours, 6 hours, or 12 hours), several days (e.g., 1 day, 2 days, 3 days, 4 days, 5 days, or 6 days), or several weeks (e.g., 1 week, 2 weeks, 3 weeks, or 4 weeks). The time between doses can be the same or different.

The nucleic acid-based drug-loaded nanovesicles can be administered by any route that results in delivery to the lungs or other tissues. Systemic routes of administration, such as intravenous bolus single injections or continuous infusions, are suitable. More direct routes, such as intranasal administration, endobronchial administration (e.g., via intubation), and inhalation (e.g., via aerosol through the mouth or nose) are contemplated and may be more appropriate, particularly where rapid action is required. An aerosol is a suspension of liquid dispersed as small particles in a gas, and includes a fine mist or spray containing such particles. Aerosolization is the process of producing an aerosol by converting a liquid suspension into small particles or droplets. This can be done using an aerosol delivery system, such as a pressurized pack or nebulizer. Nebulizers include air jet (i.e., pneumatic), ultrasonic, and vibrating mesh nebulizers, for example, using suitable propellants such as, but not limited to, dichlorodifluoromethane, trichlorofluoromethane, dichlorotetrafluoroethane, carbon dioxide, or other suitable gases. In addition to nebulizers, other devices for pulmonary delivery include, but are not limited to, metered dose inhalers (MDI) and dry powder inhalers (DPI). Capsules and cartridges, e.g., of gelatin, for use in inhalers or blowers can be formulated with lyophilized nucleic acid-based drug-loaded nanovesicles and a suitable powder base, e.g., lactose or starch.

The nucleic acid-based drug-loaded nanovesicles may be formulated for parenteral administration by injection, including large single injections or continuous infusions, when systemic delivery is desirable. Injectable formulations may be provided in unit dosage forms, e.g., ampoules or multi-dose containers, with or without preservatives. The composition may take the form of an aqueous suspension, solution, or emulsion in an oily or aqueous vehicle, and may contain a formulating agent, such as a suspending agent, stabilizing agent, and/or dispersing agent. Suitable lipophilic solvents or vehicles include fatty oils, such as sesame oil, or synthetic fatty acid esters, such as ethyl oleate or triglycerides. The aqueous injection suspension may contain a material that increases the viscosity of the suspension, such as sodium carboxymethyl cellulose, sorbitol, or dextran. Optionally, the suspension may also contain a suitable stabilizing agent, or an agent that increases solubility. Alternatively, the nucleic acid-based drug-loaded nanovesicles may be in lyophilized or other powder or solid form for composition with a suitable vehicle prior to use, e.g., sterile distilled water for injection.

It should be understood that other agents administered to a subject treated according to the present disclosure may be administered by any suitable route, including oral administration, intranasal administration, endobronchial administration, inhalation, intravenous administration, and the like. One of ordinary skill in the art will be aware of conventional routes of administration for such secondary agonists.

Nanovesicles loaded with nucleic acid-based drugs may be used immediately or, alternatively, may be stored for short and/or long periods of time, for example, cryopreserved prior to use. Typically, protease inhibitors are included in the freezing medium because they provide nanovesicle integrity during long-term storage. Freezing below -20°C is undesirable because it is associated with an increased loss of nanovesicle activity. Quick freezing below - 80°C is more preferable as it preserves activity. To enhance the preservation of the biological activity of nanovesicles loaded with nucleic acid-based drugs, adjuvants to the freezing medium can be used. These adjuvants are similar to those used for cryopreservation of intact cells and may include, but are not limited to, DMSO, glycerol, and polyethylene glycol.

Pharmaceutical preparations of the present invention are typically formulated for parenteral administration, i.e., bolus, intravenous, and intratumoral injections, in a unit-dose injectable form with a pharmaceutically acceptable parenteral vehicle. Cell-derived natural or artificial nanovesicles loaded with a nucleic acid-based drug having a desired degree of purity are randomly mixed with pharmaceutically acceptable diluents, carriers, excipients, or stabilizers to form lyophilized preparations or aqueous solutions.

### ADVANTAGEOUS EFFECTS

According to the present invention, natural or artificial nanovesicles loaded with antisense oligonucleotides inhibit the proliferation and migration of cancer cells and increase their drug sensitivity when administered in combination with conventional chemotherapeutic agents. In addition, cell-derived natural or artificial nanovesicles can be produced at a relatively low cost while utilizing the strengths of cellular therapeutics, and are expected to target cancer cells through a different mechanism than conventional immuno-oncology drugs.

### DESCRIPTION OF THE DRAWINGS

FIG. 1 is a graph analyzing the decrease in Galectin-3 gene expression in a lung cancer cell line (A549) upon Galectin-3 ASO treatment.
FIG. 2 is a graph analyzing the decrease in Galectin-3 protein expression in a lung cancer cell line (A549) upon Galectin-3 ASO treatment.
FIG. 3 is a graph showing reduced cell proliferation in a lung cancer cell line upon Galectin-3 ASO treatment and a photograph taken after staining the cells to confirm inhibition of cell migration.
FIG. 4 is a photograph of dead cells after staining to confirm that co-administration of Galectin-3 ASO with the anticancer drug Cisplatin promotes cancer cell death and increases sensitivity to the anticancer drug.
FIG. 5 is a photograph taken after analyzing Galectin-3 ASO loaded into nanovesicles by electroporation and cell extrusion using a porous membrane to determine the degree of load and efficiency.
FIG. 6 is a graph of the results of a method for loading Galectin-3 ASO in nanovesicles and then purifying the unloaded ASO to obtain pure ASO-loaded nanovesicles.
FIG. 7 is a graph showing that treatment with Galectin-3 ASO-loaded artificial nanovesicles reduced Galectin-3 gene expression in a lung cancer cell line (A549).
FIG. 8 is a photograph taken after staining the proliferated cells to confirm that the cell proliferation ability of lung cancer cells was reduced after treatment with Galectin-3 ASO-loaded umbilical cord blood stem cell exosomes and artificial nanovesicles; and a graph showing the cell proliferation ability of lung cancer cells after treatment with Galectin-3 ASO-loaded exosomes and artificial nanovesicles.
FIG. 9 is a photograph taken after staining migrated cells to confirm the reduced cell migration ability of lung cancer cells after treatment with Galectin-3 ASO-loaded umbilical cord blood stem cell exosomes and artificial nanovesicles; and a graph showing the cell migration ability of lung cancer cells after treatment with Galectin-3 ASO-loaded exosomes and artificial nanovesicles.
FIG. 10 is a photograph taken after staining the proliferated cells to confirm the reduced cell proliferation ability of lung cancer cells after treatment with Galectin-3 ASO-loaded HEK293 cell exosomes and artificial nanovesicles; and a graph showing the cell proliferation ability of lung cancer cells after treatment with Galectin-3 ASO-loaded exosomes and artificial nanovesicles.
FIG. 11 is a photograph taken after staining the migrated cells to confirm the reduced cell migration ability of lung cancer cells after treatment with Galectin-3 ASO-loaded HEK293 cell exosomes and artificial nanovesicles; and a graph showing the cell migration ability of lung cancer cells after treatment with Galectin-3 ASO-loaded exosomes and artificial nanovesicles.
FIG. 12 is a schematic illustration of the mechanism of ASOs that bind to and degrade the mRNA of target genes to induce loss of gene function (Science 27 Mar 2020; vol.367, Issue 6485).
FIG. 13 illustrates a 4 billion year old lipid bilayer shield that prevents RNA from entering.

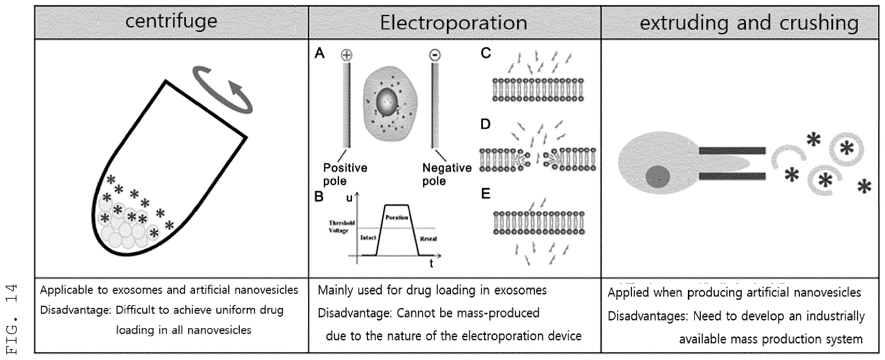

FIG. 14 is a diagram summarizing the advantages and disadvantages of different methods of drug delivery in nanovesicles.
FIG. 15 is a schematic diagram illustrating the structure and function of the respiratory system.
FIG. 16 is a schematic illustrating the structure of alveoli.
FIG. 17 illustrates the function of galectin, which is highly expressed in lung cancer cells and promotes cancer cell proliferation and metastasis (Chang WA, 2017, Oncology Letters).
FIG. 18 is a result of identifying accumulation sites in the body after intravenous administration of extracellular vesicles (Sci Rep. 2019 Jul 11;9(1):10041).

### mode for carrying out the invention

Hereinafter, the present invention will be described in more detail through examples. However, the following examples are intended to clearly illustrate the technical features of the present invention and do not limit the scope of protection of the present invention.

### Example 1. Making a Galectin-3 ASO

To construct an ASO targeting Galectin-3, three ASO sequences containing 20 consecutive bases per site within the coding sequence of Galectin-3 were randomly selected. To target Galectin-3, we designed ASOs using each sequence from SEQ ID Nos. 1 to 3, and then added phosphorothioate and 2'-O-methyl groups to the base of each sequence to increase the intracellular uptake and stability of the ASOs.

**[Table 1]**

| **No.** | **Target site** |
|---|---|
| Site 1 (SEQ ID NO. 1) | cat gat gcg tta tct ggg tc |
| Site 2 (SEQ ID NO. 2) | ggc cac tga ttg tgc ctt at |
| Site 3 (SEQ ID NO. 3) | act ggg gaa ggg aag aaa ga |

Preliminary experiments with each of the Galectin-3 ASOs designed with SEQ ID Nos. 1-3 have confirmed that all three ASOs have the same function in terms of reducing Galectin-3 expression. Therefore, in the following examples, the results of experiments with the ASO designed with SEQ ID No. 1 are illustrated as representative.

In addition, since the sequences of SEQ ID Nos. 1 to 3 were randomly selected from three sites of the entire Galectin-3 coding sequence, it is possible to produce Galectin-3 ASOs by utilizing sequences of sites other than SEQ ID Nos. 1 to 3 of the entire Galectin-3 coding sequence.

### Example 2. Loading of Galectin-3 ASO into nanovesicles

### 2-1) By electroporation

Exosomes isolated from the cell culture of umbilical cord blood stem cells were purified and concentrated using an ultracentrifuge (50,000~150,000 × g, 1-3 h). Exosomes were counted by Nanoparticle Tracking Analysis and 1-4×10^10 exosomes were diluted in 100 µl of Invitrogen Neon^{™} R buffer. To 100 µl of the prepared exosomes, 100 to 1,000 pmol of Galectin-3 ASO was added. Electroporation transfection was performed using an Invitrogen Neon^{™} Electroporator with the following conditions: 100 ul of exosomes, 1000-2000 V, 5-20 ms, and 1-3 pulses. Residual ASO was removed by IZON qEV original size exclusion chromatography (SEC) purification.

### 2-2) Extruder

The umbilical cord blood stem cells in culture were collected using TrypLE and washed twice with PBS. After cell counting, a cell suspension was prepared by resuspension in PBS at 1~10×10^5 cells/ml. Galectin-3 ASO was added to the cell suspension to reach 100-1,000 pmol/ml. Using an Avanti mini Extruder, 1 ml of the cell suspension was extruded using a 0.1 to 0.8 um Whatman^{™} Membrane Filter. Cells were extruded and ground by a total of 2 to 10 reciprocations. The extruded and ground samples were purified and concentrated using an ultracentrifuge (50,000-150,000 × g, 1-3 h). Residual ASO was removed by IZON qEV original size exclusion chromatography (SEC) purification.

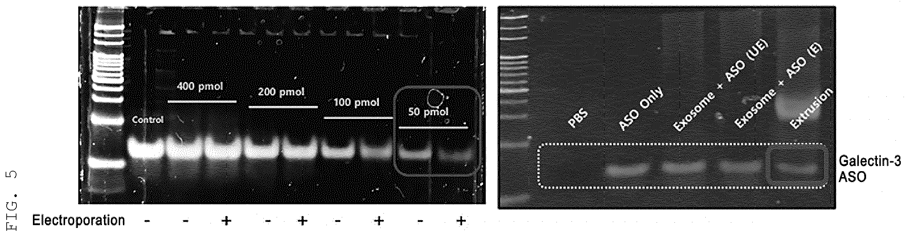

To confirm the incorporation of Galectin-3 ASO into exosomes and artificial nanovesicles, the residual ASO before and after electroporation and before and after exclusion was subjected to electrophoresis and the bands were checked, and the bands were reduced in thickness in the experimental groups, confirming the incorporation of ASO into the nanovesicles (FIG. 5).

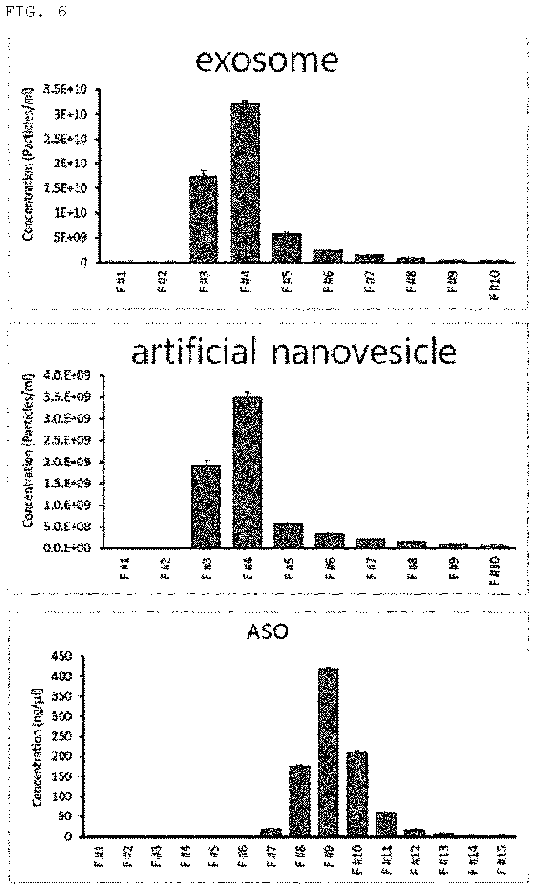

Isolation and purification of exosomes and artificial nanovesicles loaded with Galectin-3 ASO confirmed that obtaining samples between 3 and 6 fractions during the SEC process resulted in purely purified exosomes and artificial nanovesicles without residual ASO (FIG. 6).

### Example 3. Lung cancer cell line culture

A549 cells, a non-small cell lung cancer cell line, were cultured in RPMI 1640 medium supplemented with 10% fetal bovine serum (FBS), Primocin^{™} (100 ug/ml) in a 37°C, 5% CO₂ incubator. Media was changed every 2-3 days and passaged when cells had grown to 80% of the bottom of the culture dish.

### Example 4. Galectin-3 Gene Expression Analysis in Lung Cancer Cells After Galectin-3 ASO Treatment

A549 cells were seeded at 4,000 to 8,000 cells/cm² in culture dishes and incubated for 24 hours in a 37°C, 5% CO₂ incubator. 100 to 1,000 pmol Galectin-3 ASO was transfected into the cells using Lipofectamine. After 24-48 hours of incubation in a 37°C, 5% CO₂ incubator, cells were washed with PBS and harvested using TrypLE. The cells were centrifuged to remove the supernatant, and the cell pellet was resuspended in PBS and centrifuged to remove the supernatant.

Total RNA was isolated and quantified using the PureLinkTM RNA Mini Kit (Invitrogen) to synthesize cDNA.

The synthesized A579 cDNA was subjected to Real-Time PCR with the primers listed in Table 2 to determine the expression of Galectin-3. GAPDH, a kind of housekeeping gene, was used as a loading control.

**[Table 2]**

| **Gene** | **Primer Sequence** |
|---|---|
| Galectin-3 | F- GTCCGGAGCCAGCCAAC |
| | R- AGGCCATCCTTGAGGGTTTG |
| GAPDH | F- GGGTGTGAACCATGAGAA |
| | R- GTCTTGGGGTGGCAGTGAT |

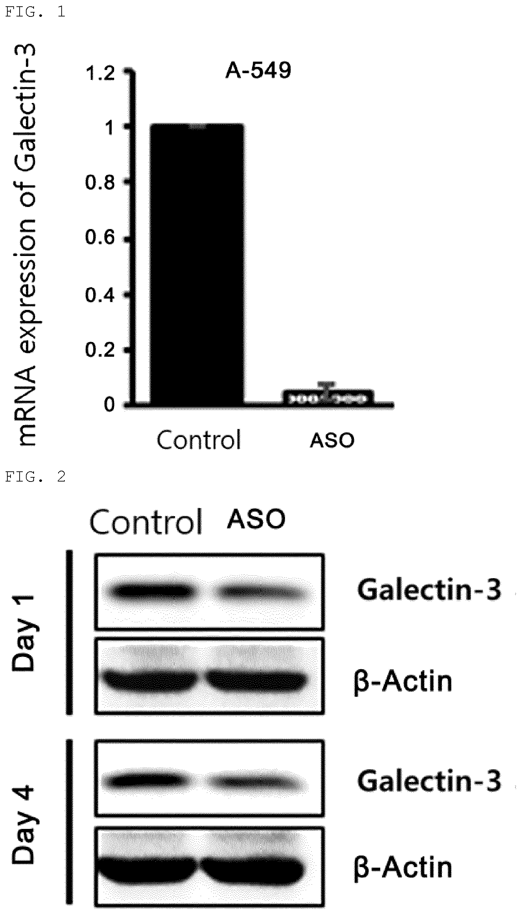

To determine whether Galectin-3 ASO made from the sequence in Table 1 works normally to reduce the gene expression of Galectin-3 in lung cancer cells, lung cancer cells were treated with ASO and analyzed using the sequence in Table 2, and it was confirmed that Galectin-3 gene expression in lung cancer cell line A549 was reduced by Galectin-3 ASO treatment (FIG. 1).

### Example 5. Analysis of Galectin-3 protein expression in lung cancer cells after Galectin-3 ASO treatment

After obtaining lung cancer cells treated with Galectin-3 ASO in the same way as in Example 4, a western blot was performed to determine the protein expression of Galectin-3 in the cells. Cell pellets were lysed with cell lysis solution (200 ng/ml phenylmethylsulfony fluoride, 1% Triton X-100, 150 mM NaCl, 50 mM Tris) containing phosphatase inhibitor cocktail (Sigma) to induce intracellular protein elution. After electrophoresis on a 10% SDS polyacrylamide gel, the lysates were transferred to an Immobilon P membrane. The membrane was then probed with Galectin-3 antibody, and the Galectin-3 band was detected by color development using an ECL kit.

The analysis showed that Galectin-3 protein expression in lung cancer cell line A549 was reduced by Galectin-3 ASO treatment (FIG. 2).

It may take several hours to several days for the effect to be seen at the protein level after the reaction at the RNA level and finally at the protein level, and in some cases, the ASO-induced decrease is seen within a short period of time (less than 24 hours) and then returns to its original state. FIG. 2 shows the level of protein expression on the 1st and 4th day after ASO treatment, and shows that Galectin-3 ASO treatment inhibited protein expression for more than 4 days and for a long period of time, confirming that it is performing a sufficient function as a drug.

### Example 6. Lung cancer cell proliferation assay after Galectin-3 ASO treatment

A549 cells were seeded at 4,000 to 8,000 cells/cm² in culture dishes and incubated for 24 hours in a 37°C, 5% CO₂ incubator. After removing all existing culture medium, 100 to 1,000 pmol Galectin-3 ASO was transfected into the cells using Lipofectamine in 1% FBS medium. Cells were counted after 24 and 48 hours of incubation in a 37°C, 5% CO₂ incubator.

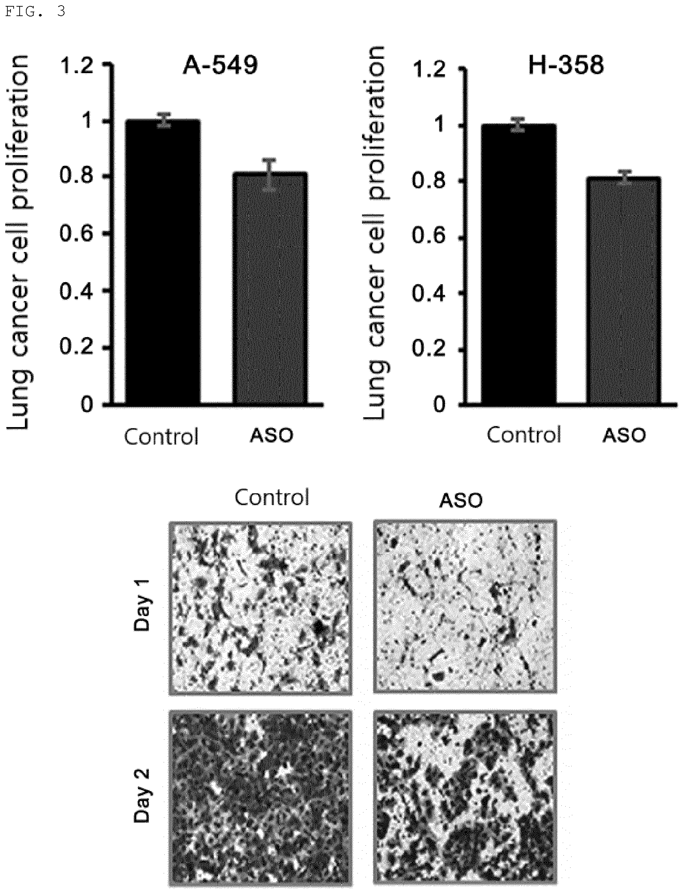

The results showed that the cell proliferation ability of lung cancer cell line A549 was reduced by Galectin-3 ASO treatment (FIG. 3 left). In the case of Example 5 (FIG. 2), the decrease in protein expression by Galectin-3 ASO treatment was confirmed, indicating that the decrease in cell proliferation and migration was induced as a result of the decrease in protein expression.

The graph in FIG. 3 shows a relative comparison of the degree of cell proliferation, which is not a quantification of the number of cells, but a comparison of the degree of cell proliferation in the experimental groups with the degree of proliferation in the control group as "1".

On the other hand, H-358 is one of the same NSCLC cell lines as A549, and as shown in FIG. 3, Galectin-3 ASO showed the same efficacy in other NSCLC cell lines, not just A549 cell lines, indicating that Galectin-3 ASO can be utilized across NSCLC.

### Example 7. Lung cancer cell migration assay after Galectin-3 ASO treatment

A549 cells were seeded in trans-well insert wells at 0.5-2 x 10^4 cells/well and incubated for 24 hours in a 37°C, 5% CO₂ incubator. After removing all existing culture medium, 100-1,000 pm Galectin-3 ASO was transfected into the cells using Lipofectamine under serum free medium conditions. After 2 hours of incubation in a 37°C, 5% CO₂ incubator, 500 µl of RPMI 1640 medium supplemented with 10% FBS, Primocin^{™} (100 ug/ml) was dispensed onto the bottom plate of the insert wells. After 48 hours of incubation at 37°C, 5% CO₂ incubator, crystal violet staining was performed. Cells that migrated to the other side of the insert well membrane were observed under a light microscope.

The results confirmed that the cell migration ability of lung cancer cell line A549 was reduced by Galectin-3 ASO treatment (FIG. 3 right).

As shown in FIG. 3, the migration of lung cancer cells was inhibited up to the second day after Galection-3 ASO treatment.

### Example 8. Analysis of cancer cell sensitivity to anticancer drugs after Galectin-3 ASO treatment

A549 lung cancer cells were attached to culture dishes at a cell count of 2 to 10 x 10^4 cells/cm² and then Galectin-3 ASO was transfected into the cells using Lipofectamine at a concentration of 100 to 1,000 pmol. After 24 hours of treatment with 10-50 uM Cisplatin, the degree of cell death was determined by fluorescence microscopy using the Live & Dead Assay Kit 24 hours later.

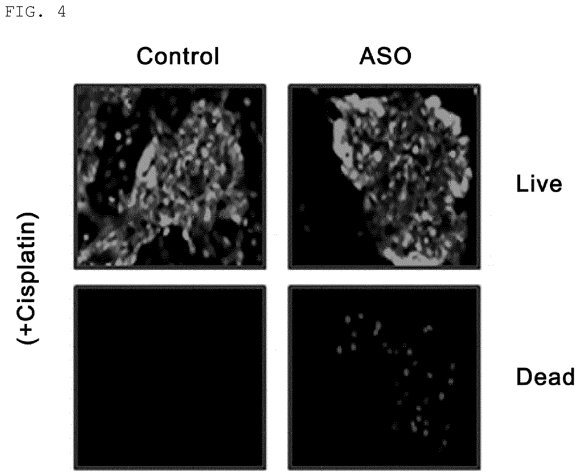

The results showed that co-administration of Galectin-3 ASO with conventional chemotherapeutic anticancer agents increased the apoptosis of lung cancer cell line A549, resulting in increased sensitivity to chemotherapeutic agents (FIG. 4).

### Example 9. Analysis of Galectin-3 gene expression in lung cancer cells after treatment with Galectin-3 ASO-loaded artificial nanovesicles

A549 cells were seeded at 4,000 to 8,000 cells/cm² in culture dishes and incubated for 24 hours in a 37°C, 5% CO₂ incubator. The next day, they were treated with 0.5 to 2 x 10^9 artificial nanovesicles loaded with Galectin-3 ASO. After 24 hours of incubation at 37°C, 5% CO₂ incubator, Galectin-3 gene expression was analyzed as in Example 4.

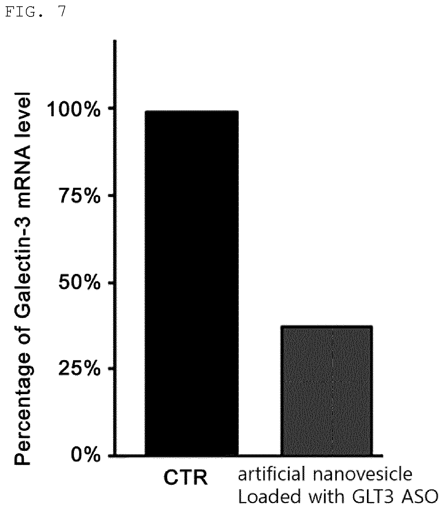

The analysis confirmed that Galectin-3 gene expression in the lung cancer cell line A549 was reduced by treatment with Galectin-3 ASO-loaded artificial nanovesicles (FIG. 7).

FIG. 1 shows the extent of intracellular Galectin-3 gene expression after transfection of Galectin-3 ASO, without nanovesicles, into cells using lipofectamine reagent. Lipofectamine is a reagent that artificially induces liposome formation and allows substances to enter cells, but it is not possible to utilize it in actual drug production due to its inherent toxicity and processing problems. Therefore, the results in FIG. 1 are a first confirmation that Galectin-3 ASO has a normal function, and in FIG. 7, Galectin-3 ASO is loaded into artificial nanovesicles and naturally enters cells without treatment with reagents such as lipofectamine, and the efficacy of inhibiting the expression of Galectin-3 is confirmed.

### Example 10: Effect of Umbilical cord blood stem cell exosomes and artificial nanovesicles without or with Galectin-3 ASO on proliferation and migration of lung cancer cell line (A549)

### (Description of experimental groups)

Control : No treatment (Negative control)
Exosome : Umbilical cord blood stem cell exosome treatment group
GAL-3 Exosome : Galectin-3 ASO-loaded umbilical cord blood stem cell exosome treatment group
ANV : Unloaded umbilical cord blood stem cell artificial nanovesicle treatment group
GAL-3 ANV : Galectin-3 ASO-loaded umbilical cord blood stem cell artificial nanovesicle treatment group

### 10-1. Lung cancer cell proliferation assay after treatment with Galectin-3 ASO-loaded nano-vesicles

A549 cells were seeded at 4,000-8,000 cells/cm² in culture dishes and cultured for 24 hours in a 37°C, 5% CO₂ incubator. After removing all existing culture medium, 0.5-2 x 10^9 exosomes and artificial nanovesicles were treated with 1% FBS medium. Cells were counted after 24 and 48 hours of incubation in a 37°C, 5% CO₂ incubator.

The results showed that the proliferation of lung cancer cell line A549 was reduced by Galectin-3 ASO-loaded nanovesicles treatment (FIG. 8). The graph in FIG. 8 shows a comparison of relative cell proliferation rates.

As shown in the graph of exosomes/artificial nanovesicles in FIG. 8, we found that Galectin-3 ASO was equally effective when loaded into both exosomes and artificial nanovesicles made by crushing cells.

### 10-2. Lung cancer cell migration assay after treatment with Galectin-3 ASO-loaded nano-vesicles

A549 cells were seeded into trans-well insert wells at 0.5-2 x 10^4 cells/well and incubated for 24 hours in a 37°C, 5% CO₂ incubator. After removing all existing culture medium, 0.5-2 x 10^9 exosomes and artificial nanovesicles were treated with serum free medium. After 2 hours of incubation at 37°C, 5% CO₂ incubator, 500 µl of RPMI 1640 medium supplemented with 10% FBS, Primocin^{™} (100 ug/ml) was dispensed on the bottom plate of the insert wells. After 48 hours of incubation at 37°C, 5% CO₂ incubator, crystal violet staining was performed. Cells that migrated to the other side of the insert well membrane were observed under a light microscope.

The results confirmed that the migration ability of lung cancer cell line A549 was reduced by treatment with Galectin-3 ASO-loaded artificial nanovesicles prepared in Example 2 (FIG. 9).

### (Conclusion)

In the case of umbilical cord blood stem cell exosomes and artificial nanovesicles without Galectin-3 ASO, they promote the proliferation and migration of lung cancer cell lines in accordance with the inherent properties of stem cells to promote the proliferation and migration of surrounding cells. However, umbilical cord blood stem cell exosomes and artificial nanovesicles carrying Galectin-3 ASO offset the inherent properties of stem cells (promoting cell proliferation/migration) by inhibiting Galectin-3 gene expression (promoting proliferation/migration of non-small cell lung cancer), resulting in inhibition of the proliferation/migration effect compared to exosomes and artificial nanovesicles not carrying Galectin-3 (Exosome & ANV), resulting in a similar degree of lung cancer cell proliferation and migration ability to the control experimental group without any treatment.

### Example 11: Effect of HEK293 cell exosomes and artificial nanovesicles without and with Galectin-3 ASO on the proliferation and migration of a lung cancer cell line (A549)

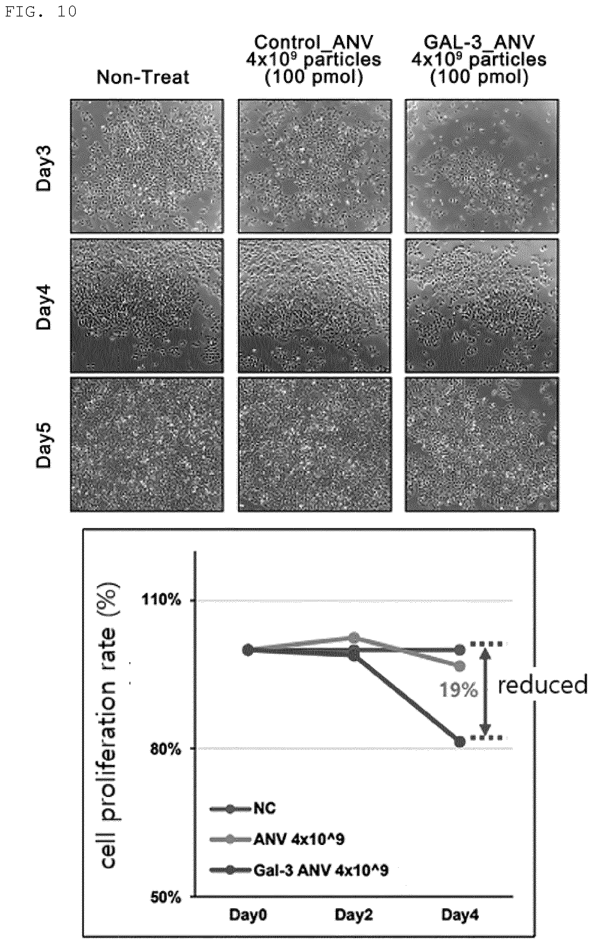

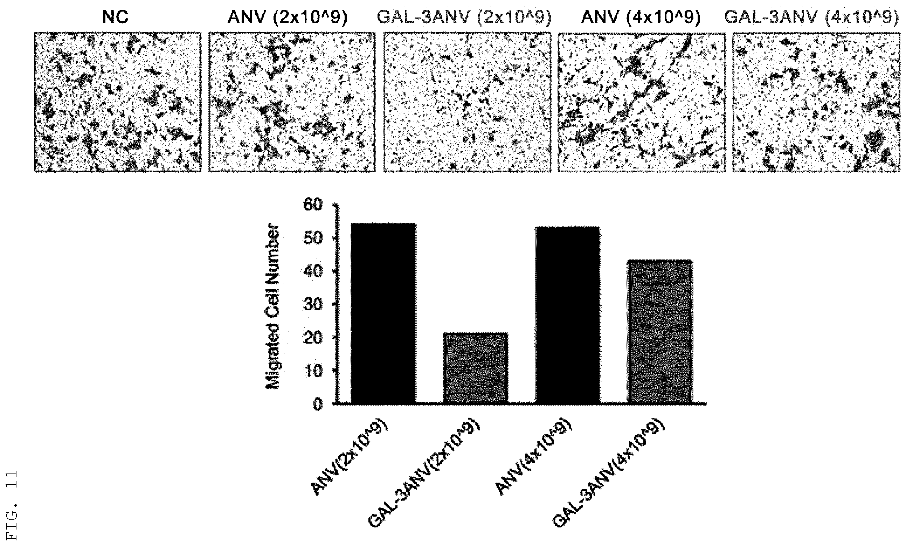

In order to exclude stem cell inherent characteristics in the effect of Galectin-3 ASO-loaded artificial nanovesicles on the proliferation and migration of lung cancer cell lines, non-stem cell HEK293 cells were utilized to produce Galectin-3 ASO-loaded artificial nanovesicles, which were then treated with lung cancer cell lines in the same manner as in Example 10 to observe the efficacy of Galectin-3 ASO. The effects on the proliferation and migration of lung cancer cell lines are shown in FIGS. 10 and 11.

### (Description of experimental groups)

Non-Treat(NC) : No treatment (Negative control)
Control_ANV: Unloaded HEK293 cell-derived artificial nanovesicles treatment group
GAL-3_ANV: Galectin-3 ASO-loaded HEK293 cell-derived artificial nanovesicles treatment group

### (Conclusion)

While the HEK293 cells-derived artificial nanovesicles without Galectin-3 ASO performed similarly to the negative control with no treatment, the HEK293 cells-derived artificial nanovesicles with Galectin-3 ASO inhibited the proliferation and migration of the lung cancer cell line. This confirms that HEK293 cell-derived artificial nanovesicles act as a carrier that can replicate the efficacy of ASOs, without affecting the HEK293 cell's own characteristics.

## Claims

**1.** A pharmaceutical composition comprising cell-derived natural or artificial nanovesicles loaded with an antisense oligonucleotide (ASO)-based drug.

**2.** A pharmaceutical composition comprising cell-derived natural or artificial nanovesicles loaded with a Galectin-3 targeting nucleic acid drug.

**3.** A pharmaceutical composition for oral or nasal inhalation or intravenous administration, comprising cell-derived natural or artificial nanovesicles loaded with a nucleic acid-based drug.

**4.** A pharmaceutical composition for the prevention or treatment of cancer, comprising umbilical cord blood stem cell-derived natural or artificial nanovesicles loaded with a nucleic acid-based drug.

**4.** The pharmaceutical composition of any one of claims 1 to 3, wherein the natural or artificial nanovesicles are released or prepared from human embryonic kidney cells or umbilical cord blood stem cells.

**6.** The pharmaceutical composition of any one of claims 1 to 4, wherein the ASO-based drug or nucleic acid-based drug is an ASO-based drug in which some or all of the bases of at least one of the sequences are modified with phosphorothioate and/or 2'-0-methyl groups.

**7.** The pharmaceutical composition of any one of claims 1 to 3, which is for the prevention or treatment of cancer.

**8.** The pharmaceutical composition of claim 1, 3 or 4, wherein the ASO-based drug or nucleic acid-based drug targets Galectin-3.

**9.** The pharmaceutical composition of any one of claims 1 to 4, wherein the ASO-based drug or nucleic acid-based drug is engineered using one or more sequences of SEQ ID NOs 1 to 3 to target Galectin-3.

**10.** The pharmaceutical composition of claim 1, 2 or 4, which is a formulation for oral or nasal inhalation or for intravenous administration.

**11.** The pharmaceutical composition of any one of claims 1 to 4, which is for the prevention or treatment of lung cancer.

**12.** The pharmaceutical composition of any one of claims 1 to 4, which is co-administered with a non-nucleic acid based drug.

**13.** The pharmaceutical composition of any one of claims 1 to 4, which reduces the proliferative and/or migratory capacity of the target cells.

**14.** The pharmaceutical composition of any one of claims 1 to 4, which increases susceptibility to co-administered anticancer drugs.

**15.** A Galectin-3 targeting antisense oligonucleotide (ASO), which is designed using a sequence within the Galectin-3 coding sequence (CDS).

**16.** The Galectin-3 targeting antisense oligonucleotide (ASO) of claim 15, which is designed using one or more sequences of SEQ ID NOs 1 to 3 to target Galectin-3.
